# EUROPEAN PATENT SPECIFICATION

(11) **EP 4 071 463 B1**
(45) Date of publication and mention of the grant of the patent: **13.03.2024**
(21) Application number: 21812605.0
(22) Date of filing: 13.05.2021
(51) Int. Cl.: G01N 33/52, B01L 3/00, G01N 21/78, G01N 21/84, G01N 31/22, G01N 21/77

(54) **REACTION TEST PAPER, DETECTION CHIP AND DETECTION SYSTEM**
REAKTIONSTESTPAPIER, DETEKTIONSCHIP UND DETEKTIONSSYSTEM
PAPIER TEST, PUCE DE DÉTECTION ET SYSTÈME DE DÉTECTION

(30) Priority: 29.05.2020 CN 202010478331
(43) Date of publication of application: 12.10.2022
(73) Proprietor: BOE Technology Group Co., Ltd., Beijing 100015 (CN); Beijing Boe Health Technology Co., Ltd., Beijing 100176 (CN)
(72) Inventor: ZHANG, Lin, Beijing 100176 (CN); SHEN, Xiaohe, Beijing 100176 (CN); SONG, Jiwen, Beijing 100176 (CN); CHANG, Xin, Beijing 100176 (CN); HU, Yasai, Beijing 100176 (CN); ZHAO, Jing, Beijing 100176 (CN)
(74) Representative: Maiwald GmbH
(86) International application number: PCT/CN2021/093635
(87) International publication number: WO 2021/238670

(56) References cited:
- EP-A1- 2 120 048
- WO-A1-2020/049561
- CN-A- 1 501 067
- CN-A- 101 261 270
- CN-A- 110 412 027
- CN-U- 206 038 690
- US-A- 4 503 145
- US-A- 4 587 102
- US-A- 4 587 102

## Description

This application claims priority of the Chinese Patent Application No. 202010478331.1, filed on May 29, 2020, the entire disclosure of which is incorporated herein by reference as part of the disclosure of this application.

### TECHNICAL FIELD

Embodiments of the present disclosure relate to a reaction test strip, a detection chip, and a detection system.

### BACKGROUND

As a conventional detection method, test strip detection is widely used in different application scenarios such as home, healthcare, scientific research, etc. The test strip detection may be to detect the content or concentration of a certain component in a solution to be detected by pre-impregnating a test strip with a detection reagent, and the test strip detection has advantages of small sample consumption, fast analysis speed, small size, portability, etc., and therefore can meet different actual needs such as instant testing, on-site analysis, or the like.
US4587102A discloses a dry type multilayer analysis element for assaying a concentration of a specific component utilizing a competitive immunological reaction. The multilayer analysis element comprises a detection layer with two or more layers composed of a fibrous porous medium with water-retention property and absorbs an amount of a sample solution necessary for the competitive immunological reaction, so that the multilayer analysis element has high sensitivity and high reproducibility. WO2020/049561A1 discloses devices, test strip and methods for testing breastmilk and providing feedback for improving the quality thereof.
EP2120048A1 discloses a strip-assembled immunochromatographic test strip plate for multiple detections. The plate includes a base, an upper cover engaged with the base, and a drainage piece disposed between the strips on the base and the upper cover, a sample-adding opening disposed on the upper cover is directly opposite to the drainage piece, and the sample-adding opening connects with a drainage groove formed by many drainage channels on underside of the upper cover. Several strip stages on the base correspond with the drainage channels on the upper cover according to their location and number, and the edge of the drainage piece laps to the sample pads adjacent to one end of the sample-adding opening.

### SUMMARY

At least one embodiment of the present disclosure provides a reaction test strip, a detection chip, and a detection system, which may solve one or more problems in the art.

The object is achieved by the features of the respective independent claims. Further embodiments are defined in the respective dependent claims.

### BRIEF DESCRIPTION OF THE DRAWINGS

In order to clearly illustrate the technical solutions of the embodiments of the present disclosure, the drawings of the embodiments will be briefly described in the following. It is obvious that the described drawings are only related to some embodiments of the present disclosure and thus are not limitative to the present disclosure.
FIG 1 is a schematic structural diagram of an example of a reaction test strip;
FIG 2A and FIG 2B are schematic structural diagrams of a reaction test strip provided by at least one embodiment of the present disclosure;
FIG 3A and FIG 3B are schematic structural diagrams of another reaction test strip provided by at least one embodiment of the present disclosure;
FIG 4A and FIG 4B are respectively a front perspective view and a rear perspective view of a detection chip provided by at least one embodiment of the present disclosure;
FIG 5 is an exploded view of the detection chip illustrated in FIG 4A and FIG 4B;
FIG 6 is a rear perspective view of a first substrate of the detection chip illustrated in FIG 4A and FIG 4B;
FIG 7 is a rear perspective view of a first substrate of another detection chip provided by at least one embodiment of the present disclosure; and
FIG 8 is a schematic block diagram of a detection system provided by at least one embodiment of the present disclosure.

### DETAILED DESCRIPTION

In order to make objects, technical solutions, and advantages of the embodiments of the present disclosure apparent, the technical solutions of the embodiments of the present disclosure will be described in a clearly and fully understandable way in connection with the drawings related to the embodiments of the present disclosure. Apparently, the described embodiments are just a part but not all of the embodiments of the present disclosure. Based on the described embodiments of the present disclosure herein, those skilled in the art can obtain other embodiment(s), without any inventive work, which should be within the scope of the present disclosure.

Unless otherwise defined, all the technical and scientific terms used herein have the same meanings as commonly understood by one of ordinary skill in the art to which the present disclosure belongs. The terms "first," "second," etc., which are used in the present disclosure, are not intended to indicate any sequence, amount or importance, but distinguish various components. Also, the terms such as "a," "an," etc., are not intended to limit the amount, but indicate the existence of at least one. The terms "comprise," "comprising," "include," "including," etc., are intended to specify that the elements or the objects stated before these terms encompass the elements or the objects and equivalents thereof listed after these terms, but do not preclude the other elements or objects. The phrases "connect," "connected," "coupled," etc., are not intended to define a physical connection or mechanical connection, but may include an electrical connection, directly or indirectly. "On," "under," "right," "left," and the like are only used to indicate relative position relationship, and when the position of the object which is described is changed, the relative position relationship may be changed accordingly.

At present, when a test strip is used for detection, due to different properties of a solution to be detected, it is often hard to ensure that the solution to be detected can fully infiltrate the test strip, which makes it difficult for the solution to be detected to fully mix and react with the reagent in the test strip, thereby reducing accuracy and reliability of the detection result. In addition, in order to infiltrate the test strip with the solution to be detected, a long detection time is often required, thereby reducing the detection efficiency and making it difficult to meet requirements of real-time detection.

A reaction test strip includes a base film layer structure and a detection reagent, the base film layer structure has water absorbency and includes a detection layer, a material of the detection layer includes cotton fiber or glass fiber, and the detection reagent is distributed in the detection layer and is configured to change a color of the base film layer structure after reacting with a sample to be detected.

The reaction test strip can help the sample to be detected quickly and sufficiently infiltrate the base film layer structure, so as to facilitate the sample to be detected reacting quickly and sufficiently with the detection reagent distributed in the detection layer, which thus can shorten the detection time and improve the accuracy and reliability of the obtained detection result, thereby improving the detection efficiency, facilitating achieving the actual needs of real-time detection, and further improving the repeatability of the detection method using the reaction test strip. In addition, by using the detection reagent that can change the color of the base film layer structure after reacting with the sample to be detected, the observation and detection of subsequent detection results can be facilitated.

For example, the reaction test strip can be used in cooperation with a detection chip, and for example, can be accommodated in a detection groove of the detection chip. In the case that the reaction test strip provided by at least one embodiment of the present disclosure is applied to a detection chip, for example, a microfluidic detection chip, since the base film layer structure of the reaction test strip has water absorbency, the fluidity of the sample to be detected in the detection chip can be further improved, so as to help the detection chip to complete the entire process of analyzing and detecting the sample to be detected through the capillary action, thereby achieving automated and integrated detection, reducing possible manual errors in the detection process, and further improving the accuracy and precision of the detection result.

The reaction test strip provided by the present disclosure will be described below through several specific embodiments.

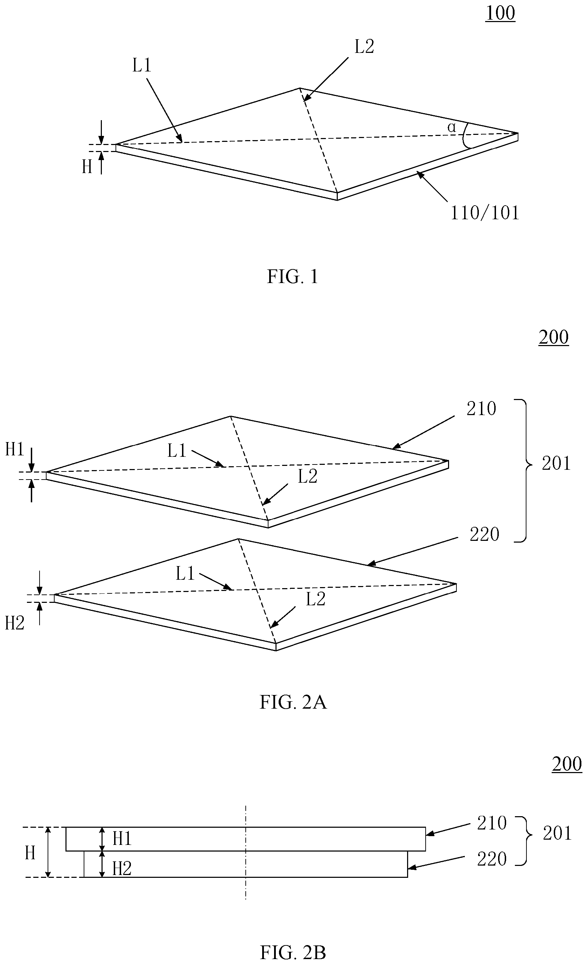

FIG 1 is a schematic structural diagram of an example of a reaction test strip, not being part of the invention.

As illustrated in FIG 1, a reaction test strip 100 includes a base film layer structure 101 and a detection reagent. The base film layer structure 101 has water absorbency and includes a detection layer 110, a material of the detection layer 110 includes cotton fiber or glass fiber, and the detection reagent is distributed in the detection layer 110 and is configured to change a color of the base film layer structure 101 after reacting with a sample to be detected.

In the reaction test strip 100, since the base film layer structure 101 has water absorbency, the amount of the sample to be detected that infiltrates the base film layer structure 101 within a certain period of time may be increased, so as to help the sample to be detected quickly and sufficiently infiltrate the base film layer structure 101 and react quickly and sufficiently with the detection reagent distributed in the detection layer 110, which thus can shorten the detection time and improve the accuracy of the obtained detection result, thereby improving reproducibility of the detection method using the reaction test strip 100. In addition, since the material having water absorbency usually also has good hydrophilicity, the reaction test strip 100 may further help the sample to be detected more uniformly and sufficiently infiltrate the base film layer structure 101, thereby further improving the accuracy of the obtained detection result.

In the reaction test strip 100, by adopting the detection reagent which may change the color of the base film layer structure 101 after reacting with the sample to be detected, it may facilitate subsequent detection of a change in the color of the base film layer structure 101 through, for example, an optical detection device, so as to achieve detection and analysis of the sample to be detected, such as presence or absence of a certain component in the sample to be detected or the amount of the content in the sample to be detected, etc., thereby achieving detection of a certain index of the sample to be detected. In addition, by using the material such as white or light-colored cotton fiber or glass fiber as the detection layer, it may facilitate subsequent detection of a change in the color of the base film layer structure 101, thereby further improving the accuracy of the detection result.

For example, in some examples, the sample to be detected may be a liquid, for example, a breast milk sample. During a preparation process of the reaction test strip 100, the required detection reagent (e.g., a chromogenic reagent) may be pre-titrated on the base film layer structure 101 of the initial reaction test strip 100, or the base film layer structure 101 may be infiltrated in the solution containing the detection reagent, so as to allow the detection reagent to be sufficiently infiltrate into the base film layer structure 101. After the detection reagent infiltrates the base film layer structure 101, the reaction test strip 100 is then dried and pressed, and for example, pressing duration may be determined according to the specific size, shape, or the like of the base film layer structure 101, so that the surface of the reaction test strip 100 may have a certain flatness, for example, the surface may be intuitively determined to be a flat surface, so as to alleviate or avoid unevenness, warpage, or the like of the surface of the reaction test strip 100. Thus, it is favorable for storage and transportation of the reaction test strip 100, and when the reaction test strip 100 is placed in the detection groove of the detection chip, a gap between the surface of the reaction test strip 100 and an internal structure of the detection chip may also be alleviated or avoided, thereby alleviating or avoiding liquid trapping or cavitation caused thereby.

For example, according to different indexes required to be detected of the sample to be detected, the detection reagent in the reaction test strip 100 includes different detection substances accordingly. After the detection reagent and the sample to be detected are mixed and react with each other, the base film layer structure 101 correspondingly reflects different changes in color. Thus, analysis and detection of a certain index in the sample to be detected may be implemented by detecting a change in color of the base film layer structure 101.

The color of the material of the base film layer structure includes white. For example, the base film layer structure 101 may use a white film material or other suitable film materials that may reflect a change in color, for example, light-colored film materials such as light yellow, light blue, etc., which may allow the color rendering effect of the base film layer structure 101 to be more obvious.

The detection reagent is used to detect a content of protein in the sample to be detected, and an initial color of the base film layer structure 101 under action of the detection reagent is green; or the detection reagent is used to detect a content of calcium in the sample to be detected, and an initial color of the base film layer structure 101 under action of the detection reagent is purple; or the detection reagent is used to detect a content of zinc in the sample to be detected, and an initial color of the base film layer structure 101 under action of the detection reagent is yellow; or the detection reagent is used to detect a content of lactose in the sample to be detected, and an initial color of the base film layer structure 101 under action of the detection reagent is white; or the detection reagent is used to detect a content of fat in the sample to be detected, and an initial color of the base film layer structure 101 under action of the detection reagent is white or shift to yellow. For example, the shift to yellow color may be a color of a yellowish system such as faint yellow, light yellow, cream, beige, or the like.

For example, after the detection reagent used for detecting the content of protein in the sample to be detected reacts with the sample to be detected, a color of the base film layer structure 101 changes to dark green. For example, after the detection reagent used for detecting the content of calcium in the sample to be detected reacts with the sample to be detected, a color of the base film layer structure 101 changes to purplish grey. For example, after the detection reagent used for detecting the content of zinc in the sample to be detected reacts with the sample to be detected, a color of the base film layer structure 101 changes to reddish orange. For example, after the detection reagent used for detecting the content of lactose in the sample to be detected reacts with the sample to be detected, a color of the base film layer structure 101 changes to blue or purplish red. For example, after the detection reagent used for detecting the content of fat in the sample to be detected reacts with the sample to be detected, a color of the base film layer structure 101 changes to blue or purplish red.

For example, taking that the reaction test strip 100 is used to detect and analyze nutrients in the breast milk sample as an example, when the reaction test strip 100 is used to detect a protein index (e.g., the protein content or concentration) in the breast milk sample, the initial color of the base film layer structure 101 under the action of the detection reagent used for detecting the protein index is green, and after the detection reagent and the breast milk sample are mixed and react with each other, the color of the base film layer structure 101 is dark green. When the reaction test strip 100 is used to detect a calcium index (e.g., the calcium content or concentration) in the breast milk sample, the initial color of the base film layer structure 101 under the action of the detection reagent used for detecting the calcium index is purple, and after the detection reagent and the breast milk sample are mixed and react with each other, the color of the base film layer structure 101 is purplish grey. When the reaction test strip 100 is used to detect a zinc index (e.g., the zinc content or concentration) in the breast milk sample, the initial color of the base film layer structure 101 under the action of the detection reagent used for detecting the zinc index is yellow, and after the detection reagent and the breast milk sample are mixed and react with each other, the color of the base film layer structure 101 is reddish orange. When the reaction test strip 100 is used to detect a lactose index (e.g., the lactose content or concentration) in the breast milk sample, the initial color of the base film layer structure 101 under the action of the detection reagent used for detecting the lactose index is white, and after the detection reagent and the breast milk sample are mixed and react with each other, the color of the base film layer structure 101 is purplish red or blue. When the reaction test strip 100 is used to detect a fat index (e.g., the fat content or concentration) in the breast milk sample, the initial color of the base film layer structure 101 under the action of the detection reagent used for detecting the fat index is white or shift to yellow, and after the detection reagent and the breast milk sample are mixed and react with each other, the color of the base film layer structure 101 is purplish red or blue.

For example, as illustrated in FIG 1, the base film layer structure 101 includes a single-layer film structure, the single-layer film structure is the detection layer 110 having water absorbency, and the detection reagent is distributed in the detection layer 110. That is, the base film layer structure 101 is composed of only one detection layer 110, and the sample to be detected is detected and analyzed through the only one detection layer 110. Thus, the preparation cost of the reaction test strip 100 may be reduced, and the preparation process of the reaction test strip 100 may be simplified, so as to be favorable for large-scale production and application.

For example, a saturated water absorption (e.g., a liquid storage amount) of the detection layer 110 ranges from 10 µL to 50 µL, for example, 30 µL. The detection layer 110 may accommodate a certain amount of sample, and after the saturated water absorption of the detection layer 110 is exceeded, the detection layer 110 will no longer absorb the sample, thereby playing a role of sample quantification, which is favorable for controlling the amount of the sample to be detected, so as to implement quantitative detection of the sample to be detected. In addition, the detection layer 110 may also reduce the amount of the sample to be detected that needs to be supplied, and reduce consumption of the sample to be detected, thereby reducing or avoiding waste of the sample, and further facilitating implementing multiple detections on the same batch of the sample to be detected, so as to further improve the accuracy of the obtained detection result.

For example, a thickness H of the detection layer 110 ranges from 0.1 mm to 0.5 mm, so as to help the sample to be detected infiltrate into the detection layer 110 uniformly and sufficiently along the surface of the detection layer 110, to sufficiently react with the detection reagent in the detection layer 110, thereby improving the accuracy of the obtained detection result.

For example, as illustrated in FIG 1, a planar shape of the base film layer structure 101 may be a rhombus, that is, a planar shape of the detection layer 110 is a rhombus. The rhombus has a long diagonal L1 and a short diagonal L2, for example, a ratio of the long diagonal L1 to the short diagonal L2 of the rhombus ranges from 1: 1 to 2:1, two obtuse inner angles and two acute inner angles are formed between the long diagonal L1 and the short diagonal L2, and the acute inner angle α of the rhombus ranges from 40° to 90°, for example, 50° or 60°, so as to help the sample to be detected infiltrate along the surface of the base film layer structure 101, for example, infiltrate from the periphery to the center of the base film layer structure 101, so as to further improve the infiltration effect of the sample to be detected, and improve the accuracy of the detection result. For example, the sample to be detected may gradually infiltrate the reaction test strip 100 from the acute inner angle of the rhombus, so as to help the sample to be detected quickly fill the entire reaction test strip 100 along the periphery of the reaction test strip 100, so that the infiltration effect of the sample to be detected is more uniform, which may allow the sample to be detected to sufficiently react with the detection reagent in the reaction test strip 100, and further improve the accuracy of the obtained detection result.

For example, the long diagonal L1 of the rhombus may range from 1 mm to 20 mm, and further for example, may range from 5 mm to 9 mm; and the short diagonal L2 of the rhombus may range from 1 mm to 20 mm, and further for example, may range from 4 mm to 8 mm. Due to the small size of the reaction test strip 100, the reaction test strip 100 can be easily stored or carried, and can be further applied to different practical application scenarios such as home, medical treatment, or the like. For example, the reaction test strip 100 may applied to a microfluidic detection chip, so as to facilitate achieving thin or miniaturized design of the overall shape of the microfluidic detection chip, thereby achieving a portable detection system.

It should be noted that, in the example illustrated in FIG 1, the reaction test strip 100 is in a rhombus shape, that is, the planar shape of the reaction test strip 100 is a rhombus; and in some other examples, the planar shape of the reaction test strip may be a regular shape such as a circle, an ellipse, a square, a rectangle, a triangle, a hexagon, etc., or may also be a suitable irregular shape or the like.

For example, a material of the detection layer 110 may include glass fiber, cotton fiber, composite fiber of glass fiber and cotton fiber, or the like, for example, cotton wool fiber or bonded glass fiber, or may also include other suitable materials having water absorbency, for example, a material having water absorbency of 25 mg/cm² to 100 mg/cm². Thus, it is favorable for the detection reagent or the sample to be detected to more uniformly and sufficiently infiltrate into the detection layer 110 and improve the infiltration effect, thereby improving uniformity of the detection effect (e.g., a color rendering effect) of the detection layer 110.

In some embodiments of the present disclosure, the base film layer structure of the reaction test strip may also include a multi-layer film structure, that is, the base film layer structure of the reaction test strip may be composed of a plurality of film layers.

FIG 2A and FIG 2B are schematic structural diagrams of a reaction test strip according to the present invention. For example, FIG 2A and FIG 2B show an example of a film layer structure of a reaction test strip including two film layers.

In some embodiments of the present disclosure, as illustrated in FIG 2A and FIG 2B, the detection layer includes a first detection layer 210 and a second detection layer 220, the base film layer structure 201 of the reaction test strip 200 includes a multi-layer film structure, and the multi-layer film structure includes the first detection layer 210 and the second detection layer 220 which are stacked with each other. For example, the first detection layer 210 and the second detection layer 220 are at least partially stacked with each other in a thickness direction. For example, the first detection layer 210 and the second detection layer 220 may be in direct contact with each other as illustrated in FIG 2B.

The detection reagent includes a first detection reagent and a second detection reagent, the first detection reagent is distributed in the first detection layer 210, the second detection reagent is distributed in the second detection layer 220, and a detection substance included in the first detection reagent is different from a detection substance included in the second detection reagent, so as to prevent the first detection reagent and the second detection reagent from reacting with each other before the detection of the sample to be detected, so that the stability of the reaction test strip 200 may be improved, thereby improving the accuracy of the obtained detection result.

For example, in the process of preparing the reaction test strip 200, the first detection reagent and the second detection reagent respectively infiltrate the first detection layer 210 and the second detection layer 220, so that the first detection reagent may be only distributed in the first detection layer 210 and not distributed in the second detection layer 220, and the second detection reagent is only distributed in the second detection layer 220 and not distributed in the first detection layer 210. After the first detection reagent and the second detection reagent sufficiently infiltrate the first detection layer 210 and the second detection layer 220, respectively, the first detection layer 210 and the second detection layer 220 are respectively dried and pressed. Therefore, the stability of the reaction test strip 200 can be improved, which is favorable for long-term storage of the reaction test strip 200, so as to further improve the accuracy of the detection result obtained with the reaction test strip 200.

For example, when the reaction test strip 200 is used for detection, after the sample to be detected infiltrates the first detection layer 210 and the second detection layer 220, the first detection reagent in the first detection layer 210 and the second detection reagent in the second detection layer 220 may be driven to flow, so that the first detection reagent, the second detection reagent, and the sample to be detected are mixed and react together to detect the sample to be detected.

For example, one of the detection substances in the first detection reagent and the second detection reagent may be a detection substance that provides a catalytic effect, so that when the reaction test strip 200 is used for detection, the mixing reaction speed of the first detection reagent, the second detection reagent, and the sample to be detected may be accelerated, thereby reducing the detection time and improving the detection efficiency.

According to the invention the first detection layer 210 and the second detection layer 220 have water absorbency, and the saturated water absorption of the first detection layer 210 is less than the saturated water absorption of the second detection layer 220, so that, as driven by the sample to be detected, the first detection reagent, the second detection reagent, and the sample to be detected can more sufficiently infiltrate into the second detection layer 220, so as to facilitate the first detection reagent, the second detection reagent, and the sample to be detected to be more uniformly and sufficiently mixed and react in the second detection layer 220.

For example, when the reaction test strip 200 is used for a detection chip, the first detection layer 210 and the second detection layer 220 may be stacked and arranged in a detection groove of the detection chip, and the second detection layer 220 with a greater saturated water absorption may be in contact with a bottom surface of the detection groove, thereby further promoting sufficient mixing of the first detection reagent, the second detection reagent, and the sample to be detected in the second detection layer 220.

For example, a total saturated water absorption of the first detection layer 210 and the second detection layer 220 ranges from 10 µL to 50 µL, the saturated water absorption of the first detection layer 210 accounts for 10% to 50% of the total saturated water absorption, and the saturated water absorption of the second detection layer 220 accounts for 50% to 90% of the total saturated water absorption. In this way, on the premise of reducing the amount of the sample to be detected, the first detection reagent and the sample to be detected in the first detection layer 210 may be further promoted to flow more sufficiently to the second detection layer 220, so as to more sufficiently and uniformly infiltrate the second detection layer 220, thereby further improving the accuracy of the obtained detection result.

For example, the materials of the first detection layer 210 and the second detection layer 220 may include glass fiber, cotton fiber, composite fiber of glass fiber and cotton fiber, or the like, for example, cotton wool fiber or bonded glass fiber, or may also include other suitable materials having water absorbency, for example, a material having water absorbency of 25 mg/cm² to 100 mg/cm². Therefore, it is favorable for the first detection reagent, the second detection reagent, or the sample to be detected to infiltrate the first detection layer 210 and the second detection layer 220 more uniformly and sufficiently, which improves the infiltration effect, thereby improving uniformity of the detection effect (e.g., a color rendering effect) of the reaction test strip 200.

According to the invention, as illustrated in FIG 2A and FIG 2B, the first detection layer 210 and the second detection layer 220 have the same planar shape, and a size of the planar shape of the first detection layer 210 is greater than a size of the planar shape of the second detection layer 220, so as to facilitate the sample to be detected to infiltrate along the periphery of the second detection layer 220, thereby further accelerating the infiltration speed of the sample to be detected in the second detection layer 220, shortening the detection time, and also promoting the sample to be detected to more sufficiently to infiltrate toward the center of the second detection layer 220, which makes the color rendering effect of the center of the second detection layer 220 more obvious, so that the accuracy of the detection result can be further improved.

For example, a single-edge size ratio of the planar shape of the second detection layer 220 to the planar shape of the first detection layer 210 ranges from 0.8:1 to 0.95:1, for example, 0.9:1, so as to further improve the infiltration effect of the sample to be detected in the second detection layer 220 and improve the accuracy of the detection result. For example, when the single-edge size ratio of the planar shape of the second detection layer 220 to the planar shape of the first detection layer 210 is 0.9:1, the infiltration effect of the sample to be detected in the first detection layer 210 and the second detection layer 220 may better meet actual needs, so that the sample to be detected can be sufficiently and uniformly react with the first detection reagent distributed in the first detection layer 210 and the second detection reagent distributed in the second detection layer 220, to further improve the accuracy of the obtained detection result.

It should be noted that, the single-edge size ratio refers to a ratio of one side in the planar shape of the first detection layer 210 to a corresponding side in the planar shape of the second detection layer 220. For example, a ratio of an area of the planar shape of the second detection layer 220 to an area of the planar shape of the first detection layer 210 may be a square of the above-mentioned single-edge size ratio.

For example, as illustrated in FIG 2A, the first detection layer 210 and the second detection layer 220 are of a rhombus shape, a long diagonal L1 of the rhombus of the first detection layer 210 ranges from 5 mm to 9 mm, further for example, 6 mm to 8 mm, a short diagonal L2 of the rhombus of the first detection layer 210 ranges from 5 mm to 8 mm, further for example, 6 mm to 7 mm, a long diagonal L1 of the rhombus of the second detection layer 220 ranges from 4 mm to 8 mm, further for example, 5 mm to 7 mm, and a short diagonal L2 of the rhombus of the second detection layer 220 ranges from 4 mm to 7 mm, further for example, 5 mm to 6 mm. In this case, a ratio of the long diagonal L1 of the rhombus of the second detection layer 220 to the long diagonal L1 of the rhombus of the first detection layer 210 is the above-mentioned single-edge size ratio, and a ratio of the short diagonal L2 of the rhombus of the second detection layer 220 to the short diagonal L2 of the rhombus of the first detection layer 210 is the above-mentioned single-edge size ratio.

For example, as illustrated in FIG 2B, a total thickness H of the first detection layer 210 and the second detection layer 220 ranges from 0.1 mm to 0.5 mm, that is, when the first detection layer 210 and the second detection layer 220 are stacked with each other, and surfaces of the first detection layer 210 and the second detection layer 220 are in contact with each other, an overall thickness H of the multi-layer film structure formed by the first detection layer 210 and the second detection layer 220 ranges from 0.1 mm to 0.5 mm, for example, 0.2 mm or 0.3 mm. For example, the thickness H1 of the first detection layer 210 ranges from 0.05 mm to 0.5 mm, for example, 0.2 mm or 0.3 mm, and the thickness H2 of the second detection layer 220 ranges from 0.1 mm to 0.5 mm, for example, 0.2 mm or 0.3 mm, so that on the premise that the reaction test strip 200 has a smaller thickness to facilitate the sample to be detected to respectively infiltrate along the surface of the first detection layer 210 and the surface of the second detection layer 220, it is helpful to achieve that the saturated water absorption of the second detection layer 220 is greater than the saturated water absorption of the first detection layer 210, so as to promote the first detection reagent, the second detection reagent, and the sample to be detected to uniformly and sufficiently react in the second detection layer 220 and improve the accuracy of the detection result.

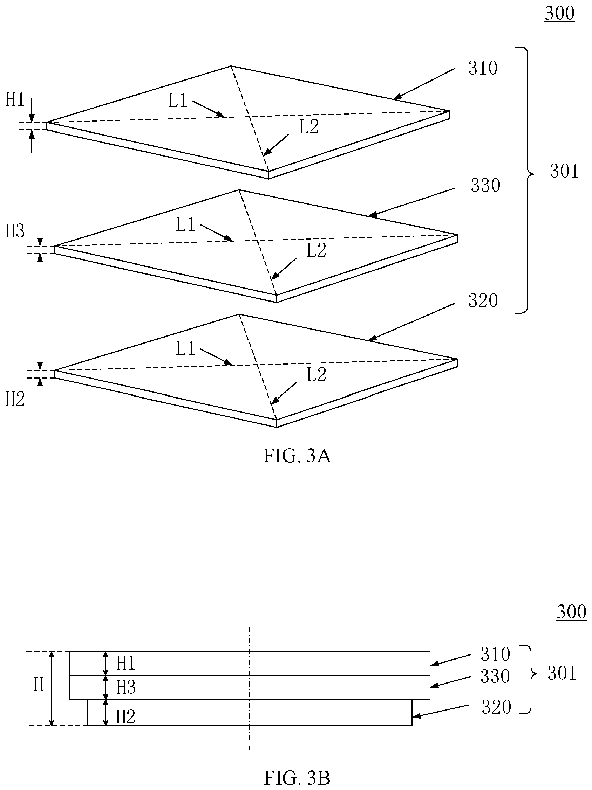

FIG 3A and FIG 3B are schematic structural diagrams of still another reaction test strip provided by at least one embodiment of the present disclosure.

For example, as illustrated in FIG 3A and FIG 3B, the base film layer structure 301 of the reaction test strip 300 includes a multi-layer film structure, the multi-layer film structure includes a water-absorbing layer 310 and a detection layer 320 which are stacked with each other, the detection reagent is distributed in the detection layer 320, and the saturated water absorption of the detection layer 320 is less than the saturated water absorption of the water-absorbing layer 310. In this way, the excessive amount of the sample to be detected in the detection layer 320 may be reduced or avoided, and the amount of the sample to be detected infiltrating the detection layer 320 may be relatively less than the amount of the sample to be detected infiltrating the water-absorbing layer 310, so that difference between different detection data can be more obvious when the reaction test strip 300 is used to detect a high concentration index of the sample to be detected, thereby further improving the accuracy of the detection result.

For example, when the reaction test strip 300 is used for the detection chip, the water-absorbing layer 310 and the detection layer 320 may be stacked in the detection groove of the detection chip, and the detection layer 320 with less saturated water absorption may be in contact with the bottom surface of the detection groove.

For example, as illustrated in FIG 3A and FIG 3B, the base film layer structure 301 further includes a hydrophilic layer 330, the water-absorbing layer 310, the hydrophilic layer 330, and the detection layer 320 are stacked in sequence, and a saturated water absorption of the hydrophilic layer 330 is less than the saturated water absorption of the water-absorbing layer 310, and is less than the saturated water absorption of the detection layer 320, so that on the premise of ensuring that the sample to be detected sufficiently infiltrates the detection layer 320, the uniformity of the infiltration of the sample to be detected in the detection layer 320 is improved, and the infiltration effect is improved, thereby further improving the accuracy of the detection result.

For example, a total saturated water absorption of the water-absorbing layer 310, the hydrophilic layer 330, and the detection layer 320 ranges from 10 µL to 50 µL, for example, 20 µL or 30 µL. The saturated water absorption of the water-absorbing layer 310 accounts for 70% to 95% of the total saturated water absorption, for example, 75%, 80% or 90%, the saturated water absorption of the detection layer 320 accounts for 5% to 30% of the total saturated water absorption, for example, 10%, 15% or 20%, and the rest is the saturated water absorption of the hydrophilic layer 330. Therefore, by controlling the saturated water absorption of the detection layer 320 and the water-absorbing layer 310, when the reaction test strip 300 is used to detect a high concentration index of the sample to be detected, the detection layer 320 may acquire the required amount of the sample to be detected, so that difference between different detection data may be more obvious, thereby further improving the accuracy of the detection result.

For example, a material of the water-absorbing layer 310 may include glass fiber, cotton fiber, composite fiber of glass fiber and cotton fiber, or the like, for example, cotton wool fiber or bonded glass fiber, or may also include other suitable water-absorbing materials, for example, a material having water absorbency of 25 mg/cm² to 100 mg/cm². A material of the hydrophilic layer 330 may include nylon, or may also include other suitable materials with low water absorbency and strong hydrophilicity. A material of the detection layer 320 may include nylon, asymmetric polyethersulfone, or asymmetric polysulfone, or may include glass fiber, cotton fiber, composite fiber of glass fiber and cotton fiber, or the like, or may also include other suitable materials with water absorbency less than that of the water-absorbing layer 310 and greater than that of the hydrophilic layer 330, so as to respectively achieve the above-mentioned saturated water absorption of the water-absorbing layer 310, the hydrophilic layer 330, and the detection layer 320.

For example, the water-absorbing layer 310, the hydrophilic layer 330, and the detection layer 320 are of the same planar shape, a size of the planar shape of the water-absorbing layer 310 is the same as a size of the planar shape of the hydrophilic layer 330, and a size of the planar shape of the detection layer 320 is less than the size of the planar shape of the water-absorbing layer 310 and the size of the planar shape of the hydrophilic layer 330. Thus, it may facilitate the sample to be detected to infiltrate along the periphery of the detection layer 320, so as to further accelerate the infiltration speed of the sample to be detected in the detection layer 320, shorten the detection time, and also promote the sample to be detected to more sufficiently infiltrate toward the center of the detection layer 320, which makes the color rendering effect of the center of the detection layer 320 more obvious, thereby further improving the accuracy of the detection result.

For example, a single-edge size ratio of the planar shape of the detection layer 320 to the planar shape of the water-absorbing layer 310 and the planar shape of the hydrophilic layer 330 ranges from 0.8:1 to 0.95:1, for example, 0.9:1, so as to further improve the infiltration effect of the sample to be detected infiltrating the detection layer 320, and improve the accuracy of the detection result. For example, when the single-edge size ratio of the planar shape of the detection layer 320 to the planar shape of the water-absorbing layer 310 and the planar shape of the hydrophilic layer 330 is 0.9:1, the infiltration effect of the sample to be detected infiltrating the water-absorbing layer 310, the hydrophilic layer 330, and the detection layer 320 may better meet actual needs, so that the sample to be detected may react more sufficiently and uniformly with the detection reagent distributed in the detection layer 320, which facilitates controlling the saturated water absorption of the detection layer 320, thereby further improving the accuracy of the obtained detection result. For example, by controlling the saturated water absorption of the detection layer 320, when the reaction test strip 300 is used to detect a high concentration index of the sample to be detected, difference between different detection data may be more obvious, thereby further improving the accuracy of the detection result.

For example, as illustrated in FIG 3A, the water-absorbing layer 310, the hydrophilic layer 330, and the detection layer 320 may be of a rhombus shape; a long diagonal L1 of the rhombus of the water-absorbing layer 310 ranges from 5 mm to 9 mm, for example, 7 mm or 8 mm; a short diagonal L2 of the rhombus of the water-absorbing layer 310 ranges from 5 mm to 8 mm, for example, 6 mm or 7 mm; a long diagonal L1 of the rhombus of the hydrophilic layer 330 ranges from 5 mm to 9 mm, for example, 7 mm or 8 mm; a short diagonal L2 of the rhombus of the hydrophilic layer 330 ranges from 5 mm to 8 mm, for example, 6 mm or 7 mm; a long diagonal L1 of the rhombus of the detection layer 320 ranges from 4 mm to 8 mm, for example, 6 mm or 7 mm; and a short diagonal L2 of the rhombus of the detection layer 320 ranges from 4 mm to 7 mm, for example, 5 mm or 6 mm. For example, as illustrated in FIG 3B, a total thickness H of the water-absorbing layer 310, the hydrophilic layer 330, and the detection layer 320 ranges from 0.1 mm to 0.5 mm, that is, when the water-absorbing layer 310, the hydrophilic layer 330, and the detection layer 320 are stacked with each other, and surfaces of the water-absorbing layer 310, the hydrophilic layer 330, and the detection layer 320 are in contact with each other, the overall thickness H of the multi-layer film structure formed by the water-absorbing layer 310, the hydrophilic layer 330, and the detection layer 320 ranges from 0.1 mm to 0.5 mm, for example, 0.2 mm or 0.3 mm. For example, the thickness H1 of the water-absorbing layer 310 may range from 0.1 mm to 0.4 mm, for example, 0.2 mm or 0.3 mm, the thickness H3 of the hydrophilic layer 330 may range from 0.05 mm to 0.2 mm, for example, 0.1 mm, and the thickness H2 of the detection layer 320 may range from 0.05 mm to 0.2 mm, for example, 0.1 mm. Therefore, on the premise that the reaction test strip 300 has a smaller thickness to facilitate infiltration of the sample to be detected along the surface of the detection layer 320, the infiltration effect of the sample to be detected infiltrating the detection layer 320 is further improved, which facilitates controlling the saturated water absorption of the detection layer 320, so that when the reaction test strip 300 is used to detect a high concentration index of the sample to be detected, difference between different detection data may be more obvious, thereby further improving the accuracy of the detection result.

In some other embodiments of the present disclosure, according to actual requirements, the multi-layer film structure of the reaction test strip may also include four layers, five layers or more film layers. For example, on the basis of the multi-layer film structure of the reaction test strip 300 illustrated in FIG 3A and FIG 3B, one or more hydrophilic layers may further be stacked between the water-absorbing layer 310 and the detection layer 320 to further improve the infiltration effect of the sample to be detected in the reaction test strip. For example, the first detection layer 210 and the second detection layer 220 in the reaction test strip 200 illustrated in FIG 2A and FIG 2B may also be used to replace the detection layer 320 in the reaction test strip 300 illustrated in FIG 3A and FIG 3B, so as to form a multi-layer film structure including four film layers. The embodiments of the present disclosure are not limited in this aspect.

At least one embodiment of the present disclosure further provides a detection chip, the detection chip includes at least one detection branch structure, and each of the at least one detection branch structure includes a detection portion and the reaction test strip according to any one of the embodiments of the present disclosure, for example, the reaction test strip 200, or the reaction test strip 300 as described in the above-mentioned embodiments. The reaction test strip may be accommodated in the detection groove, and the detection portion is configured to allow optical detection to be performed on the reaction test strip located in the detection groove.

The detection chip provided by at least one embodiment of the present disclosure may achieve integration of a plurality of functions such as mixing, analysis and detection, or the like through the reaction test strip disposed in the detection groove, and complete the whole process of analysis and detection of the sample to be detected through the active control and capillary action, so as to achieve the automated and integrated detection process, reduce manual errors that may exist in the detection process, and further improve the accuracy and precision of the detection data.

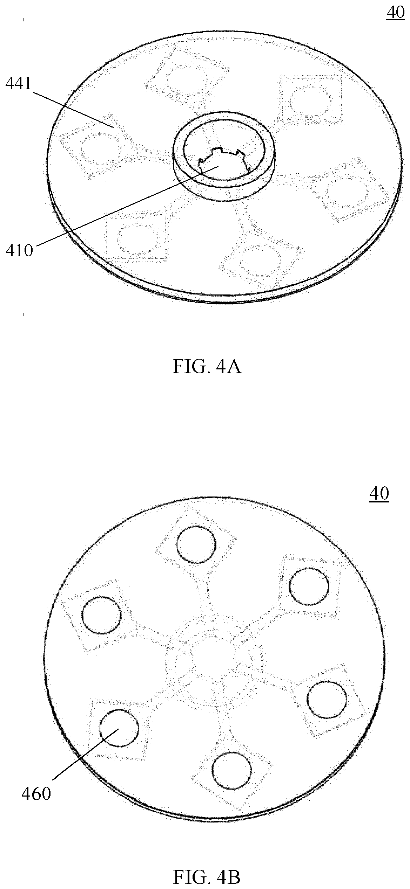

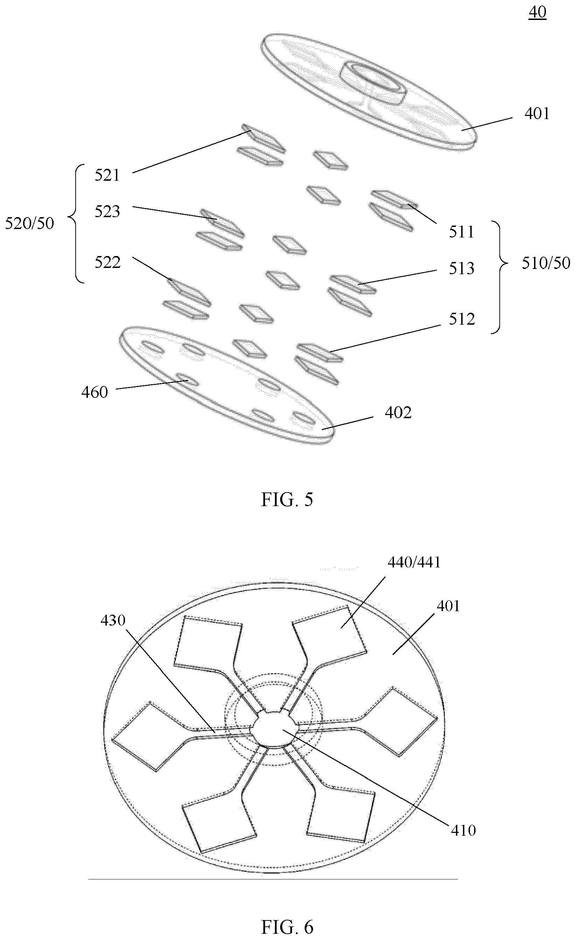

FIG 4A and FIG 4B are respectively a front perspective view and a rear perspective view of a detection chip provided by at least one embodiment of the present disclosure, FIG 5 is an exploded view, that is, a decomposition map, of the detection chip illustrated in FIG 4A and FIG 4B, and FIG 6 is a rear perspective view of a first substrate of the detection chip illustrated in FIG 4A and FIG 4B. FIG 4A is a top view of the detection chip, showing the structure viewed from the front of the detection chip; and FIG 4B is a bottom view of the detection chip, showing the structure viewed from the back of the detection chip.

For example, as illustrated in FIG 4A, FIG 4B, FIG 5 and FIG 6, the detection chip 40 includes at least one detection branch structure, for example, a plurality of detection branch structures, and six detection branch structures are illustrated in the figure as an example for introduction. The plurality of detection branch structures are uniformly distributed around the center of the detection chip 40, so that the plurality of detection branch structures may independently implement the detection function, respectively.

For example, each of the plurality of detection branch structures includes a detection portion 440. The detection portion 440 includes a detection groove 441 and a reaction test strip 50. For example, the reaction test strip 50 is substantially the same as or similar to the reaction test strip 300 according to the embodiment illustrated in FIG 3A and FIG 3B. The description of the reaction test strip 300 according to the above-mentioned embodiment may be referred to, and details are not repeated here. The reaction test strip 50 may be accommodated in the detection groove 441, and the detection portion 440 is configured to allow optical detection to be performed on the reaction test strip 50 located in the detection groove 441.

For example, as illustrated in FIG 4A, FIG 4B, FIG 5 and FIG 6, the detection chip 40 further includes a sample adding opening 410, and the sample adding opening 410 is used to add the sample to be detected, for example, the breast milk, body fluid, blood, etc. The plurality of detection grooves 441 are respectively in communication with the sample adding opening 410. Each detection branch structure further includes a guiding groove 430, the guiding groove 430 has a first end and a second end, the first end of the guiding groove 430 is in communication with the sample adding opening 410, and the second end of the guiding groove 430 is in communication with the detection groove 441, so that the detection groove 441 is in communication with the sample adding opening 410 through the guiding groove 430. Thus, the sample to be detected added from the sample adding opening 410 may enter the detection groove 441 through the guiding groove 430 and react with the detection reagent in the reaction test strip 50.

For example, the detection chip 40 further includes a first substrate 401 and a second substrate 402. The first substrate 401 has a first surface, the sample adding opening 410 is a through hole in the first substrate 401, and the guiding groove 430 and the detection groove 441 are formed on the first surface of the first substrate 401. The second substrate 402 is stacked on the first surface of the first substrate 401 and allows optical detection to be performed at a position corresponding to the detection groove 441.

For example, as illustrated in FIG 4A and FIG 4B, when the reaction test strip 50 is accommodated in the detection groove 441, the reaction test strip 50 is in a compressed state in the thickness direction. For example, a thickness of the reaction test strip 50 in a relaxed state may be equal to or slightly higher than a height of the detection groove 441, for example, a ratio of the thickness of the reaction test strip 50 in the relaxed state to the height of the detection groove 441 may range from 1:1 to 1:0.5. Thus, after combining the first substrate 401 with the second substrate 402, since the reaction test strip 50 is compressed in the thickness direction, the reaction test strip 50 is in close contact with the second substrate 402, thereby alleviating or avoiding a liquid storage phenomenon caused by a gap generated between the reaction test strip 50 and the first substrate 401 or the second substrate 402, or a bubble accumulation phenomenon due to flow of the sample to be detected, thereby further improving the accuracy of the detection result.

For example, in the embodiments of the present disclosure, six reaction test strips 50 corresponding to the six detection grooves 441 are illustrated as an example for introduction. The detection reagents in the plurality of reaction test strips 50 may be the same as each other, so that the same index in the sample to be detected can be simultaneously detected multiple times, so as to further improve the accuracy and precision of the detection result. Alternatively, the detection reagents in the plurality of reaction test strips 50 may also be different from each other, so that different detection reagents may be used to simultaneously detect a plurality of indexes in the same sample to be detected, thereby shortening a detection period of the sample and timely detecting multiple indexes in the sample.

For example, taking that the first reaction test strip 510 and the second reaction test strip 520 in the plurality of reaction test strips 50 are used to detect different indexes of the sample to be detected as an example, the first reaction test strip 510 may be used to detect the first index of the sample to be detected, the second reaction test strip 520 may be used to detect the second index of the sample to be detected, and the first detection reagent in the first reaction test strip 510 is different from the second detection reagent in the second reaction test strip 520.

For example, the first base film layer structure of the first reaction test strip 510 includes a water-absorbing layer 511, a hydrophilic layer 513, and a detection layer 512 which are stacked in sequence, and the detection layer 512 is in contact with the bottom surface of the detection groove 441. The second base film layer structure of the second reaction test strip 520 includes a water-absorbing layer 521, a hydrophilic layer 523, and a detection layer 522 which are stacked in sequence, and the detection layer 522 is in contact with the bottom surface of the detection groove 441. The first initial color of the first base film layer structure in the first reaction test strip 510 under the action of the first detection reagent is different from the second initial color of the second base film layer structure in the second reaction test strip 520 under the action of the second detection reagent. After the first detection reagent in the first reaction test strip 510 reacts with the sample to be detected, the first initial color of the first base film layer structure changes to a first color, for example, the detection layer 512 may reflect the first color. After the second detection reagent in the second reaction test strip 520 reacts with the sample to be detected, the second initial color of the second base film layer structure changes to a second color, for example, the detection layer 522 may reflect the second color. The first color is different from the second color.

For example, the first index and the second index may respectively be any two of, for example, protein, calcium, zinc, lactose, fat, etc. For example, taking that the first index is protein and the second index is fat as an example, the first detection reagent in the first reaction test strip 510 is used to detect a protein content in the sample to be detected, and the second detection reagent in the second reaction test strip 520 is used to detect a fat content in the sample to be detected. The first initial color of the first base film layer structure in the first reaction test strip 510 under the action of the first detection reagent is green, and the second initial color of the second base film layer structure in the second reaction test strip 520 under the action of the second detection reagent is white or shift to yellow, thereby distinguishing the first initial color from the second initial color. After the first detection reagent in the first reaction test strip 510 reacts with the sample to be detected, the first initial color of the first base film layer structure changes to dark green, that is, the first color reflected by the detection layer 512 is dark green. After the second detection reagent in the second reaction test strip 520 reacts with the sample to be detected, the second initial color of the second base film layer structure changes to purplish red or blue, that is, the second color reflected by the detection layer 522 is purplish red or blue. Therefore, through different color changes of the first base film layer structure and the second base film layer structure, it is favorable for distinguishing different indexes of the sample to be detected in different detection branch structures.

For example, as illustrated in FIG 4A, FIG 4B, FIG 5 and FIG 6, the planar shape of the base film layer structure of the reaction test strip 50 (e.g., the first base film layer structure of the first reaction test strip 510 and the second base film layer structure of the second reaction test strip 520) is the same as or similar to the planar shape of the detection groove 441, and the size of the planar shape of the base film layer structure is smaller than the size of the planar shape of the detection groove 441, so that the reaction test strip 50 may be stably accommodated in the detection groove 441, and it is favorable for the sample to be detected to infiltrate from the periphery of the reaction test strip 50 to the center of the reaction test strip 50 when flowing into the detection groove 441. Thus, the infiltration speed of the sample to be detected is accelerated, which facilitates uniform mixing between the reaction test strip 50 and the sample to be detected, and allows the color rendering effect of the reaction test strip 50 to be more obvious, so as to further improve the accuracy of the detection result.

For example, a single-edge size ratio of the planar shape of the base film layer structure of the reaction test strip 50 to the planar shape of the detection groove 441 ranges from 0.7:1 to 0.95:1, for example, 0.9:1, which not only facilitates the reaction test strip 50 to be stably accommodated in the detection groove 441, but also may promote the sample to be detected to infiltrate the reaction test strip 50 uniformly and sufficiently along the surface of the reaction test strip 50, for example, to promote the sample to be detected to infiltrate the reaction test strip 50 from the periphery of the reaction test strip 50 to the center of the reaction test strip 50, so as to improve the infiltration effect of the sample to be detected and reduce the detection time, thereby improving the detection efficiency and further improving the accuracy of the detection result.

For example, as illustrated in FIG 4A, FIG 4B, FIG 5 and FIG 6, the detection groove 441 and the base film layer structure of the reaction test strip 50 are of a rhombus shape; a ratio of a long diagonal of the rhombus of the base film layer structure of the reaction test strip 50 to a long diagonal of the rhombus of the detection groove 441 ranges from 0.7:1 to 0.95:1, for example, 0.9:1; a ratio of a short diagonal of the rhombus of the base film layer structure of the reaction test strip 50 to a short diagonal of the rhombus of the detection groove 441 ranges from 0.7:1 to 0.95:1, for example, 0.9:1; and a ratio of an acute inner angle of the rhombus of the base film layer structure to an acute inner angle of the rhombus of the detection groove 441 ranges from 0.7:1 to 1:1, for example, 0.9:1. In this way, it not only facilitates the reaction test strip 50 to be stably accommodated in the detection groove 441, but also facilitates the sample to be detected to uniformly and sufficiently infiltrate the reaction test strip 50 along the surface of the reaction test strip 50, for example, from the periphery of the reaction test strip 50 to the center of the reaction test strip 50, so as to improve the infiltration effect of the sample to be detected, promote uniform and sufficient mixing and reaction between the sample to be detected and the detection reagent, and reduce the detection time, thereby improving the detection efficiency and further improving the accuracy of the detection result.

For example, an inner wall of the guiding groove 430 may be set to be hydrophilic, for example, a contact angle of the sample to be detected on the inner wall of the guiding groove 430 is smaller than 90°, so as to facilitate the sample to be detected to quickly enter the guiding groove 430, so that the sample may quickly flow into the detection groove 441 through the guiding groove 430 to be mixed with the reaction test strip 50, thereby shortening the detection time of the sample and further improving the accuracy of the obtained detection data. For example, in the process of preparing the detection chip 40, a hydrophilic treatment agent may be injected into the guiding groove 430, so that the guiding groove 430 is infiltrated by the hydrophilic treatment agent for one minute, and then the hydrophilic treatment agent is discharged with air, so as to allow the inner wall of the guiding groove 430 to be set to have hydrophilicity. For example, the hydrophilic treatment agent may be a surfactant with a concentration of 0.0001% to 0.1%. For example, the surfactant may be a non-ionic surfactant such as the span series, Tween series, polyoxyethylene ether of alkyl phenol, etc., or may also be an amphoteric surfactant of an amino acid type or betaine type, which will not be limited by the embodiments of the present disclosure.

It should be noted that, the contact angle of the liquid on the inner wall of the guiding groove 430 refers to an angle formed by a tangent of a droplet surface of the sample to be detected and the inner wall surface of the guiding groove 430. For example, when the contact angle of the sample to be detected on the inner wall of the guiding groove 430 is less than 90°, it may be considered that the inner wall of the guiding groove 430 is hydrophilic. The less the value of the contact angle of the liquid on the inner wall of the guiding groove 430, the better the wettability of the inner wall of the guiding groove 430. For example, when the contact angle of the sample to be detected on the inner wall of the guiding groove 430 is 0°, the inner wall material of the guiding groove 430 is completely wetted. The specific value of the contact angle of the liquid on the inner wall of the guiding groove 430 will not be limited by the embodiments of the present disclosure, as long as the contact angle is less than 90° to allow the inner wall of the guiding groove 430 to be hydrophilic.

It should be noted that, in some embodiments, the second end of the guiding groove 430 (that is, the end in communication with the detection groove 441) is in contact with the reaction test strip 50, so as to facilitate the sample to infiltrate into the reaction test strip 50, further allow the sample to be detected to sufficiently react with the detection reagent in the reaction test strip 50, and improve the accuracy of the acquired detection data. For example, a part of the reaction test strip 50 may be located in the guiding groove 430, for example, one corner of the rhombus-shaped reaction test strip 50 may be in direct communication with the second end of the guiding groove 430. In some other embodiments of the present disclosure, the reaction test strip 50 may not be in contact with the second end of the guiding groove 430. The embodiments of the present disclosure are not limited in this aspect.

It should be noted that, in some other embodiments of the present disclosure, the detection chip 40 may not be provided with the guiding groove 430, for example, the detection groove 441 is directly connected with the sample adding opening 410 to implement communication, so as to further reduce or avoid sample waste during flowing. At this time, in some examples, the sample adding opening 410 may have a flow guiding structure respectively in communication with the plurality of detection grooves 441, so that the sample added into the sample adding opening 410 may flow into the plurality of detection grooves 441 uniformly.

For example, a material of the second substrate 402 may include one or more of polymethyl methacrylate, polystyrene, polycarbonate, and polyethylene terephthalate. For example, a material of the first substrate 401 may include one or more of polymethyl methacrylate, polystyrene, and polycarbonate, or may also be other materials with high light transmittance. The materials of the first substrate 401 and the second substrate 402 are not specifically limited by the embodiments of the present disclosure.

For example, the first substrate 401 and the second substrate 402 may be combined by bonding (referring to a technology in which two sheets of materials are bonded together by the Van der Waals force, molecular force, atomic force, etc.), welding, sticking, or clamping, so as to simplify the process such as processing, assembling, or the like of the detection chip 40, and reduce the preparation cost of the detection chip 40. For example, the first substrate 401 and the second substrate 402 may be combined by the process such as ultrasonic bonding, thermocompression bonding, laser welding, ultrasonic welding, silicone sealing or the like. The specific combination method between the first substrate 401 and the second substrate 402 is not limited by the embodiments of the present disclosure.

For example, a shape of the first substrate 401 may be a circle, a rectangle or other suitable shapes, and a shape of the second substrate 402 may also be a circle, a rectangle or other suitable shapes.

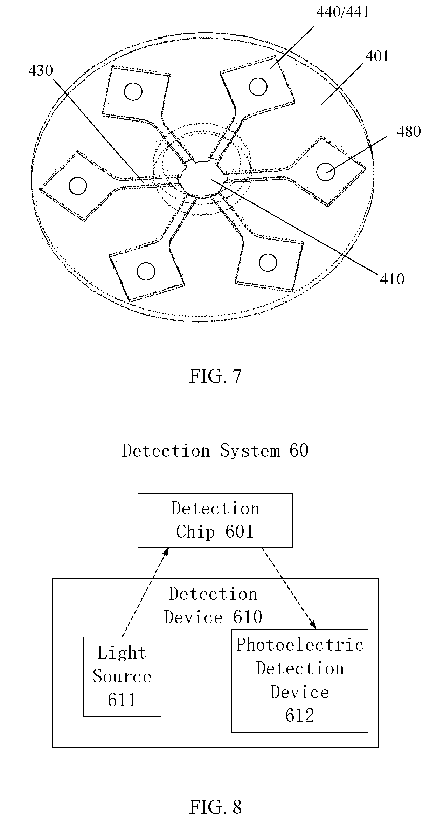

FIG 7 is a rear perspective view of a first substrate of another detection chip provided by at least one embodiment of the present disclosure. It should be noted that, other structures of the first substrate 401 illustrated in FIG 7 except for a liquid storage through hole 480 are substantially the same as those of the first substrate 401 illustrated in FIG 4A, FIG 4B, FIG 5, and FIG 6, the corresponding description in the above-mentioned embodiments illustrated in FIG 4A, FIG 4B, FIG 5, and FIG 6 may be referred to, and details are not repeated here.

For example, as illustrated in FIG 7, the detection portion of the detection chip further includes a liquid storage through hole 480 running through the first substrate 401 and being in communication with the detection groove 441, so that when the amount of the sample injected through the sample adding opening 410 is excessive, the excessive sample may be stored in the liquid storage through hole 480, so as to avoid or alleviate the leakage phenomenon caused by the injection of excessive sample. Furthermore, the liquid storage through hole 480 may also promote the sample to infiltrate from the periphery to the center of the reaction test strip, so that the detection result (e.g., color rendering) of the center of the reaction test strip may be more obvious.

For example, the liquid storage through hole 480 is in communication with the center of the detection groove 441, that is, the liquid storage through hole 480 is opened at a center position of the bottom surface of the detection groove 441, so that the center of the detection groove 441 is in communication with the external environment such as the atmosphere, thereby better facilitating the sample to infiltrate from the periphery to the center of the reaction test strip.

For example, in the preparation process of the detection chip, in order to facilitate assembly of the detection chip, a negative pressure may be added to the liquid storage through hole 480 during assembling, so as to fix the reaction test strip in the detection groove 441. Thus, the sample to be detected may react more sufficiently with the detection reagent in the reaction test strip, so as to further improve the accuracy of the obtained detection result.

It should be noted that, in some other embodiments of the present disclosure, according to the actual structure of the detection chip, the liquid storage through hole may also be located in the second substrate, that is, run through the second substrate and be in communication with the detection groove. Alternatively, in some other embodiments of the present disclosure, the liquid storage through hole may also run through the first substrate and the second substrate at the same time, and be in communication with the detection groove. The embodiments of the present disclosure are not limited in this aspect.

It should be noted that, in some other embodiments of the present disclosure, the liquid storage through hole may also be disposed at other positions, for example, in a region away from the guiding groove, so as to alleviate or avoid the possible adverse effect of unnecessary sample stored in the liquid storage through hole on subsequent detection and analysis of the reaction test strip with the optical detection device. Alternatively, according to actual needs, a plurality of liquid storage through holes may also be opened in each detection portion. The arrangement positions, the number, and the like of liquid storage through holes are not limited by the embodiments of the present disclosure.

For example, in some embodiments of the present disclosure, structures such as the sample adding opening, the guiding groove, the detection groove, or the like of the detection chip may also be arranged in other ways, as long as the arrangement of the structures such as the sample adding opening, the guiding groove, the detection groove, or the like may achieve corresponding functions. The above-mentioned embodiment is described in the case that the sample adding opening runs through the first substrate, and structures such as the guiding groove and the detection groove are provided on the first surface of the first substrate of the detection chip, but this does not constitute a limitation on the present disclosure.

At least one embodiment of the present disclosure further provides a detection system, the detection system includes a detection device and a detection chip provided by any one of the embodiments of the present disclosure, for example, the detection chip 40 according to the above-mentioned embodiment, and the detection device is configured to detect the reaction test strip in the detection groove through the detection portion of the detection chip.

FIG 8 is a schematic block diagram of a detection system provided by at least one embodiment of the present disclosure.

For example, as illustrated in FIG 8, the detection system 60 includes a detection chip 601 and a detection device 610. For example, the detection chip 601 may be a detection chip provided by any one of the embodiments of the present disclosure, for example, the detection chip 40 according to the above-mentioned embodiment. The corresponding description of the detection chip 40 according to the above-mentioned embodiment may be referred to for the specific content of the detection chip 601, and details are not repeated here.

For example, the detection device 610 is configured to detect the reaction test strip in the detection groove through the detection portion of the detection chip 601.

For example, the detection device 610 includes a light source 611 and a photoelectric detection device 612. The light source 611 is configured to emit light to the reaction test strip in the detection chip 601, and the photoelectric detection device 612 is configured to receive the light emitted by the light source 611 and reflected by the reaction test strip.

For example, the photoelectric detection device 612 may compare the intensity of the light reflected by the reaction test strip in the detection chip 601 with the intensity of the light emitted by the light source 611, so as to determine such as the presence or absence and concentration of a substance to be detected in the detected sample according to a value of light absorbance of the reaction test strip, thereby achieving the detection of the sample index.

For example, the detection device 610 may be configured to detect a change in the color of the base film layer structure of the reaction test strip in the detection groove through the detection portion. For example, the detection reagent in the reaction test strip may be a chromogenic reagent. When the detection result is obtained, the darker the color displayed on the reaction test strip, the higher the content of the substance to be detected in the detected sample, and accordingly, the greater the value of light absorbance of the reaction test strip detected with the photoelectric detection device 612.

For example, in some embodiments, the photoelectric detection device 612 may be a photodiode, and the photodiode may convert a received optical signal into an electrical signal, and then may determine an intensity of the received light according to a change in an electrical parameter in the electrical signal (e.g., a change in current, etc.), so as to determine the value of light absorbance of the reaction test strip.

The corresponding contents in the reaction test strip and the detection chip provided by the embodiments of the present disclosure may be referred to for specific description and technical effects of the detection system provided by the embodiments of the present disclosure, for example, the corresponding contents of the reaction test strip 100, the reaction test strip 200, the reaction test strip 300, or the detection chip 40 according to the above-mentioned embodiments may be referred to, and details are not repeated here.

For the present disclosure, the following statements should be noted:
(1) The accompanying drawings related to the embodiment(s) of the present disclosure involve only the structure(s) in connection with the embodiment(s) of the present disclosure, and other structure(s) can be referred to common design(s).
(2) For the purpose of clarity, in accompanying drawings for illustrating the embodiment(s) of the present disclosure, the thickness of a layer or a region may be enlarged or narrowed, that is, the drawings are not drawn in a real scale. However, it should be understood that, in the case that a component such as a layer, a film, a region, a substrate, or the like is referred to be "on" or "under" another component, the component may be "directly" "on" or "under" the another component, or an intermediate component may be disposed therebetween.
(3) In case of no conflict, the embodiments of the present disclosure and features in one embodiment or in different embodiments can be combined to obtain new embodiments.

## Claims

1. A reaction test strip (200, 300), comprising:
a base film layer structure (201) with water absorbency, comprising a detection layer (210, 220), wherein a material of the detection layer comprises cotton fiber or glass fiber; and
a detection reagent, wherein the detection reagent is distributed in the detection layer, and is configured to change a color of the base film layer structure after reacting with a sample to be detected,
wherein the detection layer comprises a first detection layer (210) and a second detection layer (220), the base film layer structure (201) comprises a multi-layer film structure, and the multi-layer film structure comprises the first detection layer (210) and the second detection layer (220) which are stacked with each other;
the detection reagent comprises a first detection reagent and a second detection reagent, the first detection reagent is distributed in the first detection layer (210), the second detection reagent is distributed in the second detection layer (220), and a detection substance comprised in the first detection reagent is different from a detection substance comprised in the second detection reagent;
the first detection layer (210) and the second detection layer (220) have water absorbency, and a saturated water absorption of the first detection layer (210) is less than a saturated water absorption of the second detection layer (220); and
a planar shape of the first detection layer (210) is identical to a planar shape of the second detection layer (220), and a size of the planar shape of the first detection layer (210) is greater than a size of the planar shape of the second detection layer (220).

2. The reaction test strip (200, 300) according to claim 1, wherein the detection reagent is used to detect a content of protein in the sample to be detected, and an initial color of the base film layer structure under action of the detection reagent is green; of
the detection reagent is used to detect a content of calcium in the sample to be detected, and an initial color of the base film layer structure under action of the detection reagent is purple; or
the detection reagent is used to detect a content of zinc in the sample to be detected, and an initial color of the base film layer structure under action of the detection reagent is yellow; or
the detection reagent is used to detect a content of lactose in the sample to be detected, and an initial color of the base film layer structure under action of the detection reagent is white; or
the detection reagent is used to detect a content of fat in the sample to be detected, and an initial color of the base film layer structure under action of the detection reagent is white or shift to yellow.

3. The reaction test strip (200, 300) according to claim 2, wherein after the detection reagent used for detecting the content of protein in the sample to be detected reacts with the sample to be detected, a color of the base film layer structure changes to dark green; or
after the detection reagent used for detecting the content of calcium in the sample to be detected reacts with the sample to be detected, a color of the base film layer structure changes to purplish grey; or
after the detection reagent used for detecting the content of zinc in the sample to be detected reacts with the sample to be detected, a color of the base film layer structure changes to reddish orange; or
after the detection reagent used for detecting the content of lactose in the sample to be detected reacts with the sample to be detected, a color of the base film layer structure changes to blue or purplish red; or
after the detection reagent used for detecting the content of fat in the sample to be detected reacts with the sample to be detected, a color of the base film layer structure changes to blue or purplish red.

4. The reaction test strip (200, 300) according to any one of claims 1-3, wherein a thickness of the detection layer ranges from 0.1 mm to 0.5 mm.

5. The reaction test strip (200, 300) according to claim 1, wherein a total saturated water absorption of the first detection layer (210) and the second detection layer (220) ranges from 10 µL to 50 µL,
the saturated water absorption of the first detection layer (210) accounts for 10% to 50% of the total saturated water absorption, and
the saturated water absorption of the second detection layer (220) accounts for 50% to 90% of the total saturated water absorption.

6. The reaction test strip (200, 300) according to claim 1, wherein a single-edge size ratio of the planar shape of the second detection layer (220) to the planar shape of the first detection layer (210) ranges from 0.8: 1 to 0.95: 1;
for example, the first detection layer (210) and the second detection layer (220) are in a rhombus shape;
a long diagonal (L1) of a rhombus of the first detection layer (210) ranges from 5 mm to 9 mm, and a short diagonal (L2) of the rhombus of the first detection layer (210) ranges from 5 mm to 8 mm; and
a long diagonal (L1) of a rhombus of the second detection layer (220) ranges from 4 mm to 8 mm, and a short diagonal (L2) of the rhombus of the second detection layer (220) ranges from 4 mm to 7 mm.

7. The reaction test strip (300) according to any one of claims 1-3, wherein the base film layer structure comprises a multi-layer film structure,
the multi-layer film structure comprises a water-absorbing layer (310) and the detection layers (210, 220) which are stacked with each other, and
a saturated water absorption of the detection layer (320) is less than a saturated water absorption of the water-absorbing layer (310).

8. The reaction test strip (300) according to claim 7, wherein the multi-layer film structure further comprises a hydrophilic layer (330),
the water-absorbing layer (310), the hydrophilic layer (330), and the detection layers (210, 220) are stacked in sequence, and
a saturated water absorption of the hydrophilic layer (330) is less than the saturated water absorption of the detection layers (210, 220),
for example, a total saturated water absorption of the water-absorbing layer (310), the hydrophilic layer (330), and the detection layers (210, 220) ranges from 10 µL to 50 µL,
the saturated water absorption of the water-absorbing layer (310) accounts for 70% to 95% of the total saturated water absorption, and
the saturated water absorption of the detection layers (210, 220) accounts for 5% to 30% of the total saturated water absorption.

9. The reaction test strip (300) according to claim 8, wherein a planar shape of the water-absorbing layer (310), a planar shape of the hydrophilic layer (330), and a planar shape of the detection layers (210, 220) are identical,
a size of the planar shape of the water-absorbing layer (310) is identical to a size of the planer shape of the hydrophilic layer (330), and
a size of the planar shape of the detection layers (210, 220) is smaller than the size of the planar shape of the water-absorbing layer (310) and is smaller than the size of the planar shape of the hydrophilic layer (330).

10. The reaction test strip (300) according to claim 9, wherein a single-edge size ratio of the planar shape of the detection layers (210, 220) to the planar shape of the water-absorbing layer (310) ranges from 0.8:1 to 0.95:1, and
a single-edge size ratio of the planar shape of the detection layers (210, 220) to the planar shape of the hydrophilic layer (330) ranges from 0.8:1 to 0.95:1.

11. The reaction test strip (200, 300) according to any one of claims 1-10, wherein the base film layer structure (101) is in a rhombus shape,
a ratio of a long diagonal (L1) to a short diagonal (L2) of a rhombus of the base film layer structure ranges from 1:1 to 2:1,
an acute inner angle (α) of the rhombus of the base film layer structure ranges from 40° to 90°,
the long diagonal (L1) of the rhombus of the base film layer structure ranges from 1 mm to 20 mm, and
the short diagonal (L2) of the rhombus of the base film layer structure ranges from 1 mm to 20 mm.

12. A detection chip (40), comprising at least one detection branch structure, wherein each of the at least one detection branch structure comprises:
a detection portion (440), comprising a detection groove (441) and a reaction test strip (200, 300) according to any of the previous claims, wherein the reaction test strip is configured to be accommodated in the detection groove (441),
wherein the detection portion (440) is configured to allow optical detection to be performed on the reaction test strip in the detection groove (441);
the reaction test strip comprises a base film layer structure and a detection reagent, the base film layer structure has water absorbency and comprises a detection layer, a material of the detection layer comprises cotton fiber or glass fiber, and the detection reagent is distributed in the detection layer and is configured to change a color of the base film layer structure after reacting with a sample to be detected;
the detection layer comprises a first detection layer and a second detection layer, the base film layer structure comprises a multi-layer film structure, and the multi-layer film structure comprises the first detection layer and the second detection layer which are stacked with each other;
the detection reagent comprises a first detection reagent and a second detection reagent, the first detection reagent is distributed in the first detection layer, the second detection reagent is distributed in the second detection layer, and a detection substance comprised in the first detection reagent is different from a detection substance comprised in the second detection reagent;
the first detection layer and the second detection layer have water absorbency, and a saturated water absorption of the first detection layer is less than a saturated water absorption of the second detection layer; and
a planar shape of the first detection layer is identical to a planar shape of the second detection layer, and a size of the planar shape of the first detection layer is greater than a size of the planar shape of the second detection layer.

## Patentansprüche

1. Reaktionsteststreifen (200, 300), umfassend:
eine Basisfilmschichtstruktur (201) mit Wasserabsorptionsfähigkeit, die eine Erfassungsschicht (210, 220) umfasst, wobei ein Material der Erfassungsschicht Baumwollfaser oder Glasfaser umfasst; und
ein Nachweisreagenz, wobei das Nachweisreagenz in der Nachweisschicht verteilt ist und so konfiguriert ist, dass es eine Farbe der Basisfilmschichtstruktur nach Reaktion mit einer nachzuweisenden Probe ändert,
wobei die Detektionsschicht eine erste Detektionsschicht (210) und eine zweite Detektionsschicht (220) umfasst, die Basisfilmschichtstruktur (201) eine mehrschichtige Filmstruktur umfasst und die mehrschichtige Filmstruktur die erste Detektionsschicht (210) und die zweite Detektionsschicht (220) umfasst, die miteinander gestapelt sind;
das Nachweisreagenz ein erstes Nachweisreagenz und ein zweites Nachweisreagenz umfasst, das erste Nachweisreagenz in der ersten Nachweisschicht (210) verteilt ist, das zweite Nachweisreagenz in der zweiten Nachweisschicht (220) verteilt ist und eine Nachweissubstanz, die in dem ersten Nachweisreagenz enthalten ist, von einer Nachweissubstanz, die in dem zweiten Nachweisreagenz enthalten ist, verschieden ist;
die erste Erfassungsschicht (210) und die zweite Erfassungsschicht (220) eine Wasserabsorptionsfähigkeit aufweisen und eine gesättigte Wasserabsorption der ersten Erfassungsschicht (210) geringer ist als eine gesättigte Wasserabsorption der zweiten Erfassungsschicht (220); und
eine ebene Form der ersten Erfassungsschicht (210) identisch mit einer ebenen Form der zweiten Erfassungsschicht (220) ist, und eine Größe der ebenen Form der ersten Erfassungsschicht (210) größer ist als eine Größe der ebenen Form der zweiten Erfassungsschicht (220).

2. Reaktionsteststreifen (200, 300) gemäß Anspruch 1, wobei das Nachweisreagenz verwendet wird, um einen Proteingehalt in der nachzuweisenden Probe nachzuweisen, und eine anfängliche Farbe der Basisfilmschichtstruktur unter Einwirkung des Nachweisreagenzes grün ist; oder
das Nachweisreagenz verwendet wird, um einen Kalziumgehalt in der nachzuweisenden Probe nachzuweisen, und eine anfängliche Farbe der Basisfilmschichtstruktur unter Einwirkung des Nachweisreagenzes violett ist; oder
das Nachweisreagenz verwendet wird, um einen Zinkgehalt in der nachzuweisenden Probe nachzuweisen, und eine anfängliche Farbe der Basisfilmschichtstruktur unter Einwirkung des Nachweisreagenzes gelb ist; oder
das Nachweisreagenz verwendet wird, um einen Laktosegehalt in der nachzuweisenden Probe nachzuweisen, und eine anfängliche Farbe der Basisfilmschichtstruktur unter Einwirkung des Nachweisreagenzes weiß ist; oder
das Nachweisreagenz verwendet wird, um einen Fettgehalt in der nachzuweisenden Probe nachzuweisen, und eine anfängliche Farbe der Basisfilmschichtstruktur unter Einwirkung des Nachweisreagenzes weiß ist oder zu gelb wechselt.

3. Reaktionsteststreifen (200, 300) gemäß Anspruch 2, wobei nach der Reaktion des zum Nachweis des Proteingehalts in der nachzuweisenden Probe verwendeten Nachweisreagenzes mit der nachzuweisenden Probe eine Farbe der Basisfilmschichtstruktur zu Dunkelgrün wechselt; oder
nachdem das zum Nachweis des Kalziumgehalts in der nachzuweisenden Probe verwendete Nachweisreagenz mit der nachzuweisenden Probe reagiert hat, sich die Farbe der Basisfilmschichtstruktur in ein violettes Grau ändert; oder
nachdem das zum Nachweis des Zinkgehalts in der nachzuweisenden Probe verwendete Nachweisreagenz mit der nachzuweisenden Probe reagiert hat, sich die Farbe der Basisfilmschichtstruktur rötlich-orange verfärbt; oder
nach der Reaktion des zum Nachweis des Laktosegehalts in der nachzuweisenden Probe verwendeten Nachweisreagens mit der nachzuweisenden Probe eine Farbänderung der Basisfilmschichtstruktur zu blau oder violettrot erfolgt; oder
nachdem das zum Nachweis des Fettgehalts in der zu detektierenden Probe verwendete Nachweisreagenz mit der zu detektierenden Probe reagiert hat, ändert sich die Farbe der Basisfilmschichtstruktur zu blau oder violettrot.

4. Reaktionsteststreifen (200, 300) gemäß einem der Ansprüche 1 bis 3, wobei die Dicke der Nachweisschicht im Bereich von 0,1 mm bis 0,5 mm liegt.

5. Reaktionsteststreifen (200, 300) gemäß Anspruch 1, wobei die gesamte gesättigte Wasserabsorption der ersten Nachweisschicht (210) und der zweiten Nachweisschicht (220) im Bereich von 10 µL bis 50 µL liegt,
die gesättigte Wasseraufnahme der ersten Detektionsschicht (210) 10 % bis 50 % der gesamten gesättigten Wasseraufnahme ausmacht, und
die gesättigte Wasseraufnahme der zweiten Nachweisschicht (220) 50 % bis 90 % der gesamten gesättigten Wasseraufnahme ausmacht.

6. Reaktionsteststreifen (200, 300) gemäß Anspruch 1, wobei das Verhältnis der Größe einer einzelnen Kante der planaren Form der zweiten Nachweisschicht (220) zur planaren Form der ersten Nachweisschicht (210) im Bereich von 0,8:1 bis 0,95:1 liegt;
die erste Erfassungsschicht (210) und die zweite Erfassungsschicht (220) beispielsweise die Form eines Rhombus aufweisen;
eine lange Diagonale (L1) eines Rhombus der ersten Erfassungsschicht (210) im Bereich von 5 mm bis 9 mm liegt, und eine kurze Diagonale (L2) des Rhombus der ersten Erfassungsschicht (210) im Bereich von 5 mm bis 8 mm liegt; und
eine lange Diagonale (L1) eines Rhombus der zweiten Erfassungsschicht (220) im Bereich von 4 mm bis 8 mm liegt, und eine kurze Diagonale (L2) des Rhombus der zweiten Erfassungsschicht (220) im Bereich von 4 mm bis 7 mm liegt.

7. Reaktionsteststreifen (300) gemäß einem der Ansprüche 1 bis 3, **dadurch gekennzeichnet, dass** die Basisfolienschichtstruktur (301) eine mehrschichtige Folienstruktur aufweist,
die mehrschichtige Filmstruktur eine wasserabsorbierende Schicht (310) und die Detektionsschichten (210, 220) umfasst, die miteinander gestapelt sind, und
eine gesättigte Wasseraufnahme der Nachweisschicht (320) geringer ist als eine gesättigte Wasseraufnahme der wasserabsorbierenden Schicht (310).

8. Reaktionsteststreifen (300) gemäß Anspruch 7, wobei die mehrschichtige Filmstruktur ferner eine hydrophile Schicht (330) umfasst,
die wasserabsorbierende Schicht (310), die hydrophile Schicht (330) und die Detektionsschichten (210, 220) nacheinander gestapelt sind, und
eine gesättigte Wasseraufnahme der hydrophilen Schicht (330) geringer ist als die gesättigte Wasseraufnahme der Nachweisschichten (210, 220);
Die gesamte gesättigte Wasseraufnahme der wasserabsorbierenden Schicht (310), der hydrophilen Schicht (330) und der Nachweisschichten (210, 220) liegt beispielsweise zwischen 10 µl und 50 µl,
die gesättigte Wasseraufnahme der wasserabsorbierenden Schicht (310) 70 % bis 95 % der gesamten gesättigten Wasseraufnahme ausmacht, und
die gesättigte Wasseraufnahme der Detektionsschichten (210, 220) macht 5 bis 30 % der gesamten gesättigten Wasseraufnahme aus.

9. Reaktionsteststreifen (300) gemäß Anspruch 8, wobei eine planare Form der wasserabsorbierenden Schicht (310), eine planare Form der hydrophilen Schicht (330) und eine planare Form der Nachweisschichten (210, 220) identisch sind,
eine Größe der ebenen Form der wasserabsorbierenden Schicht (310) identisch ist mit einer Größe der ebenen Form der hydrophilen Schicht (330), und
eine Größe der planaren Form der Erfassungsschichten (210, 220) kleiner ist als die Größe der planaren Form der wasserabsorbierenden Schicht (310) und kleiner ist als die Größe der planaren Form der hydrophilen Schicht (330).

10. Reaktionsteststreifen (300) gemäß Anspruch 9, wobei das Verhältnis der Größe einer einzelnen Kante der planaren Form der Nachweisschichten (210, 220) zur planaren Form der wasserabsorbierenden Schicht (310) im Bereich von 0,8:1 bis 0,95:1 liegt und
ein Verhältnis der Größe der einzelnen Kanten der ebenen Form der Erkennungsschichten (210, 220) zu der ebenen Form der hydrophilen Schicht (330) im Bereich von 0,8:1 bis 0,95:1 liegt.

11. Reaktionsteststreifen (200, 300) gemäß einem der Ansprüche 1-10, **dadurch gekennzeichnet, dass** die Basisfilmschichtstruktur (101) rhombusförmig ist,
ein Verhältnis einer langen Diagonale (L1) zu einer kurzen Diagonale (L2) eines Rhombus der Basisfilmschichtstruktur im Bereich von 1:1 bis 2:1 liegt,
ein spitzer Innenwinkel (α) des Rhombus der Grundschichtstruktur zwischen 40° und 90° liegt,
die lange Diagonale (L1) des Rhombus der Basisfilmschichtstruktur zwischen 1 mm und 20 mm liegt und
die kurze Diagonale (L2) des Rhombus der Grundschichtstruktur liegt zwischen 1 mm und 20 mm.

12. Erkennungschip (40), der mindestens eine Erkennungszweigstruktur umfasst, wobei jede der mindestens einen Erkennungszweigstruktur umfasst:
einen Detektionsabschnitt (440), der eine Detektionsrille (441) und einen Reaktionsteststreifen (200, 300) nach einem der vorhergehenden Ansprüche umfasst, wobei der Reaktionsteststreifen (200, 300) so konfiguriert ist, dass er in der Detektionsrille (441) aufgenommen werden kann,
wobei der Detektionsabschnitt (440) so konfiguriert ist, dass eine optische Detektion an dem Reaktionsteststreifen (200, 300) in der Detektionsrille (441) durchgeführt werden kann;
der Reaktionsteststreifen eine Basisfilmschichtstruktur und ein Nachweisreagenz umfasst, die Basisfilmschichtstruktur wasserabsorbierend ist und eine Nachweisschicht umfasst, ein Material der Nachweisschicht Baumwollfasern oder Glasfasern umfasst und das Nachweisreagenz in der Nachweisschicht verteilt ist und so konfiguriert ist, dass es eine Farbe der Basisfilmschichtstruktur nach Reaktion mit einer nachzuweisenden Probe ändert;
die Erkennungsschicht eine erste Erkennungsschicht und eine zweite Erkennungsschicht umfasst, die Basisfilmschichtstruktur eine mehrschichtige Filmstruktur umfasst, und die mehrschichtige Filmstruktur die erste Erkennungsschicht und die zweite Erkennungsschicht umfasst, die miteinander gestapelt sind;
das Nachweisreagenz ein erstes Nachweisreagenz und ein zweites Nachweisreagenz umfasst, das erste Nachweisreagenz in der ersten Nachweisschicht verteilt ist, das zweite Nachweisreagenz in der zweiten Nachweisschicht verteilt ist und eine Nachweissubstanz, die in dem ersten Nachweisreagenz enthalten ist, von einer Nachweissubstanz, die in dem zweiten Nachweisreagenz enthalten ist, verschieden ist;
die erste Erfassungsschicht und die zweite Erfassungsschicht eine Wasserabsorptionsfähigkeit aufweisen und eine gesättigte Wasserabsorption der ersten Erfassungsschicht geringer ist als eine gesättigte Wasserabsorption der zweiten Erfassungsschicht; und
eine ebene Form der ersten Erfassungsschicht identisch mit einer ebenen Form der zweiten Erfassungsschicht ist, und eine Größe der ebenen Form der ersten Erfassungsschicht größer ist als eine Größe der ebenen Form der zweiten Erfassungsschicht.

## Revendications

1. Bande de test de réaction (200, 300), comprenant :
une structure de couche de film de base (201) à absorption d'eau, comprenant une couche de détection (210, 220),
dans laquelle un matériau de la couche de détection comprend de la fibre de coton ou de la fibre de verre ; et
un réactif de détection, dans lequel le réactif de détection est réparti dans la couche de détection, et est configuré pour changer une couleur de la structure de couche de film de base après avoir réagi avec un échantillon à détecter,
dans laquelle la couche de détection comprend une première couche de détection (210) et une deuxième couche de détection (220), la structure de couche de film de base (201) comprend une structure de film multicouche, et la structure de film multicouche comprend la première couche de détection (210) et la deuxième couche de détection (220) qui sont empilées l'une sur l'autre ;
le réactif de détection comprend un premier réactif de détection et un deuxième réactif de détection, le premier réactif de détection est réparti dans la première couche de détection (210), le deuxième réactif de détection est réparti dans la deuxième couche de détection (220), et une substance de détection comprise dans le premier réactif de détection est différente d'une substance de détection comprise dans le deuxième réactif de détection ;
la première couche de détection (210) et la deuxième couche de détection (220) ont une absorption d'eau, et une absorption d'eau saturée de la première couche de détection (210) est inférieure à une absorption d'eau saturée de la deuxième couche de détection (220) ; et
une forme plane de la première couche de détection (210) est identique à une forme plane de la deuxième couche de détection (220), et une taille de la forme plane de la première couche de détection (210) est plus grande qu'une taille de la forme plane de la deuxième couche de détection (220).

2. Bande de test de réaction (200, 300) selon la revendication 1, dans laquelle le réactif de détection est utilisé pour détecter une teneur en protéine dans l'échantillon à détecter, et une couleur initiale de la structure de couche de film de base sous l'action du réactif de détection est le vert ; ou
le réactif de détection est utilisé pour détecter une teneur en calcium dans l'échantillon à détecter, et une couleur initiale de la structure de couche de film de base sous l'action du réactif de détection est le violet ; ou
le réactif de détection est utilisé pour détecter une teneur en zinc dans l'échantillon à détecter, et une couleur initiale de la structure de couche de film de base sous l'action du réactif de détection est le jaune ; ou
le réactif de détection est utilisé pour détecter une teneur en lactose dans l'échantillon à détecter, et une couleur initiale de la structure de couche de film de base sous l'action du réactif de détection est le blanc ; ou
le réactif de détection est utilisé pour détecter une teneur en matière grasse dans l'échantillon à détecter, et une couleur initiale de la structure de couche de film de base sous l'action du réactif de détection est le blanc ou passe au jaune.

3. Bande de test de réaction (200, 300) selon la revendication 2, dans laquelle une fois que le réactif de détection utilisé pour détecter la teneur en protéine dans l'échantillon à détecter réagit avec l'échantillon à détecter, une couleur de la structure de couche de film de base devient vert foncé ; ou
une fois que le réactif de détection utilisé pour détecter une teneur en calcium dans l'échantillon à détecter réagit avec l'échantillon à détecter, une couleur de la structure de couche de film de base devient gris violacé ; ou
une fois que le réactif de détection utilisé pour détecter une teneur en zinc dans l'échantillon à détecter réagit avec l'échantillon à détecter, une couleur de la structure de couche de film de base devient orange rougeâtre ; ou
une fois que le réactif de détection utilisé pour détecter une teneur en lactose dans l'échantillon à détecter réagit avec l'échantillon à détecter, une couleur de la structure de couche de film de base devient bleu ou rouge violacé ; ou
une fois que le réactif de détection utilisé pour détecter une teneur en matière grasse dans l'échantillon à détecter réagit avec l'échantillon à détecter, une couleur de la structure de couche de film de base devient bleu ou rouge violacé.

4. Bande de test de réaction (200, 300) selon l'une quelconque des revendications 1-3, dans laquelle une épaisseur de la couche de détection va de 0,1 mm à 0,5 mm.

5. Bande de test de réaction (200, 300) selon la revendication 1, dans laquelle une absorption d'eau saturée totale de la première couche de détection (210) et de la deuxième couche de détection (220) va de 10 µL à 50 µL,
l'absorption d'eau saturée de la première couche de détection (210) représente 10 % à 50 % de l'absorption d'eau saturée totale, et
l'absorption d'eau saturée de la deuxième couche de détection (220) représente 50 % à 90 % de l'absorption d'eau saturée totale.

6. Bande de test de réaction (200, 300) selon la revendication 1, dans laquelle un rapport de taille unilatérale de la forme plane de la deuxième couche de détection (220) sur la forme plane de la première couche de détection (210) va de 0,8:1 à 0,95:1 ;
par exemple, la première couche de détection (210) et la deuxième couche de détection (220) ont une forme de losange,
une diagonale longue (L1) d'un losange de la première couche de détection (210) va de 5 mm à 9 mm, et une diagonale courte (L2) du losange de la première couche de détection (210) va de 5 mm à 8 mm ; et
une diagonale longue (L1) d'un losange de la deuxième couche de détection (220) va de 4 mm à 8 mm, et une diagonale courte (L2) du losange de la deuxième couche de détection (220) va de 4 mm à 7 mm.

7. Bande de test de réaction (300) selon l'une quelconque des revendications 1-3, dans laquelle la structure de couche de film de base comprend une structure de film multicouche,
la structure de film multicouche comprend une couche d'absorption d'eau (310) et les couches de détection (210, 220) qui sont empilées les unes sur les autres, et
une absorption d'eau saturée de la couche de détection (320) est inférieure à une absorption d'eau saturée de la couche d'absorption d'eau (310).

8. Bande de test de réaction (300) selon la revendication 7, dans laquelle la structure de film multicouche comprend en outre une couche hydrophile (330),
la couche d'absorption d'eau (310), la couche hydrophile (330), et les couches de détection (210, 220) sont empilées successivement, et
une absorption d'eau saturée de la couche hydrophile (330) est inférieure à l'absorption d'eau saturée des couches de détection (210, 220) ;
par exemple, une absorption d'eau saturée totale de la couche d'absorption d'eau (310), de la couche hydrophile (330), et des couches de détection (210, 220) va de 10 µL à 50 µL,
l'absorption d'eau saturée de la couche d'absorption d'eau (310) représente 70 % à 95 % de l'absorption d'eau saturée totale, et
l'absorption d'eau saturée des couches de détection (210, 220) représente 5 % à 30 % de l'absorption d'eau saturée totale.

9. Bande de test de réaction (300) selon la revendication 8, dans laquelle une forme plane de la couche d'absorption d'eau (310), une forme plane de la couche hydrophile (330), et une forme plane des couches de détection (210, 220) sont identiques,
une taille de la forme plane de la couche d'absorption d'eau (310) est identique à une taille de la forme plane de la couche hydrophile (330), et
une taille de la forme plane des couches de détection (210, 220) est plus petite que la taille de la forme plane de la couche d'absorption d'eau (310) et est plus petite que la taille de la forme plane de la couche hydrophile (330).

10. Bande de test de réaction (300) selon la revendication 9, dans laquelle un rapport de taille unilatérale de la forme plane des couches de détection (210, 220) sur la forme plane de la couche d'absorption d'eau (310) va de 0,8:1 à 0,95:1 ; et
un rapport de taille unilatérale de la forme plane des couches de détection (210, 220) sur la forme plane de la couche hydrophile (330) va de 0,8:1 à 0,95:1.

11. Bande de test de réaction (200, 300) selon l'une quelconque des revendications 1-10, dans laquelle la structure de couche de film de base (101) a la forme d'un losange,
un rapport d'une diagonale longue (L1) sur une diagonale courte (L2) d'un losange de la structure de couche de film de base va de 1:1 à 2:1,
un angle intérieur aigu (α) du losange de la structure de couche de film de base va de 40° à 90°,
la diagonale longue (L1) du losange de la structure de couche de film de base va de 1 mm à 20 mm, et
la diagonale courte (L2) du losange de la structure de couche de film de base va de 1 mm à 20 mm.

12. Puce de détection (40), comprenant au moins une structure à branche de détection, dans laquelle chacune de la au moins une structure à branche de détection comprend :
une partie de détection (440), comprenant une rainure de détection (441) et une bande de test de réaction (200, 300) selon l'une quelconque des revendications précédentes,
dans laquelle la bande de test de réaction est configurée pour être logée dans la rainure de détection (441),
dans laquelle la partie de détection (440) est configurée pour permettre qu'une détection optique soit réalisée sur la bande de test de réaction dans la rainure de détection (441) ;
la bande de test de réaction comprend une structure de couche de film de base et un réactif de détection, la structure de couche de film de base a une absorption d'eau et comprend une couche de détection, un matériau de la couche de détection comprend de la fibre de coton ou de la fibre de verre, et le réactif de détection est réparti dans la couche de détection, et est configuré pour changer une couleur de la structure de couche de film de base après avoir réagi avec un échantillon à détecter,
la couche de détection comprend une première couche de détection et une deuxième couche de détection, la structure de couche de film de base comprend une structure de film multicouche, et la structure de film multicouche comprend la première couche de détection et la deuxième couche de détection qui sont empilées l'une sur l'autre ;
le réactif de détection comprend un premier réactif de détection et un deuxième réactif de détection, le premier réactif de détection est réparti dans la première couche de détection, le deuxième réactif de détection est réparti dans la deuxième couche de détection, et une substance de détection comprise dans le premier réactif de détection est différente d'une substance de détection comprise dans le deuxième réactif de détection ;
la première couche de détection et la deuxième couche de détection ont une absorption d'eau, et une absorption d'eau saturée de la première couche de détection est inférieure à une absorption d'eau saturée de la deuxième couche de détection ; et
une forme plane de la première couche de détection est identique à une forme plane de la deuxième couche de détection, et une taille de la forme plane de la première couche de détection est plus grande qu'une taille de la forme plane de la deuxième couche de détection.
